# EUROPEAN PATENT APPLICATION

(11) **EP 4 253 610 A1**
(43) Date of publication of application: **04.10.2023**
(21) Application number: 21897036.6
(22) Date of filing: 24.11.2021
(51) Int. Cl.: C40B 40/08, C40B 30/04

(54) **DNA-ENCODED COMPOUND LIBRARY AND COMPOUND SCREENING METHOD**

(30) Priority: 27.11.2020 CN 202011353377
(71) Applicant: Hitgen Inc., Chengdu, Sichuan 610200 (CN)
(72) Inventor: LI, Jin, Chengdu, Sichuan 610200 (CN); LIU, Guansai, Chengdu, Sichuan 610200 (CN); LUO, Huadong, Chengdu, Sichuan 610200 (CN); CHEN, Liu, Chengdu, Sichuan 610200 (CN); ZHU, Junyang, Chengdu, Sichuan 610200 (CN); MA, Huiyong, Chengdu, Sichuan 610200 (CN); SONG, Chao, Chengdu, Sichuan 610200 (CN); WAN, Jinqiao, Chengdu, Sichuan 610200 (CN)
(74) Representative: Vitina, Maruta
(86) International application number: PCT/CN2021/132846
(87) International publication number: WO 2022/111536

(57) **Abstract**

A synthesis and screening method of a DNA-encoded compound library. The DNA-encoded compound library consists of a DNA-encoded compound of formula (I). The screening method includes: incubating the DNA-encoded compound library with a protein target, followed by covalent cross-linking to obtain a covalently cross-linked complex; separating the covalently cross-linked complex from members in the library that do not bind to the protein target; and subjecting the covalently cross-linked complex to polymerase chain reaction (PCR) amplification and DNA sequencing.

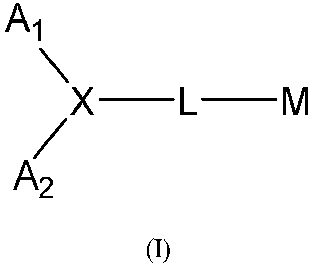

## Description

### TECHNICAL FIELD

This application relates to DNA-encoded libraries, and more particularly to a DNA-encoded compound library and a screening method thereof.

### BACKGROUND

In drug discovery, especially in new drug discovery, the high-throughput screening against biological targets is one of the main tools for rapid access to lead compounds. However, the traditional high-throughput screening based on individual molecules is time-consuming and costly in equipment. Besides, the number of compound species (millions) in a compound library is limited, and the compound library requires decades of accumulation to be established, limiting the efficiency and possibility of lead compound discovery. In recent years, DNA-encoded compound library technology has emerged, as described in, for example, international patent applications WO 2005058479 and WO 2018166532, and Chinese patent publication No. 103882532, which combines combinatorial chemistry and molecular biology technology, adds a deoxyribonucleic acid (DNA) tag to each compound at the molecular level, and enables the synthesis of libraries containing up to billions of compounds in a short time. Therefore, it is promising to be the next generation technology for compound library screening and is beginning to be widely used in the pharmaceutical industry showing many positive results, as described, for example, by Raphael (Accounts of Chemical Research, 2014, 47, 1247-1255).

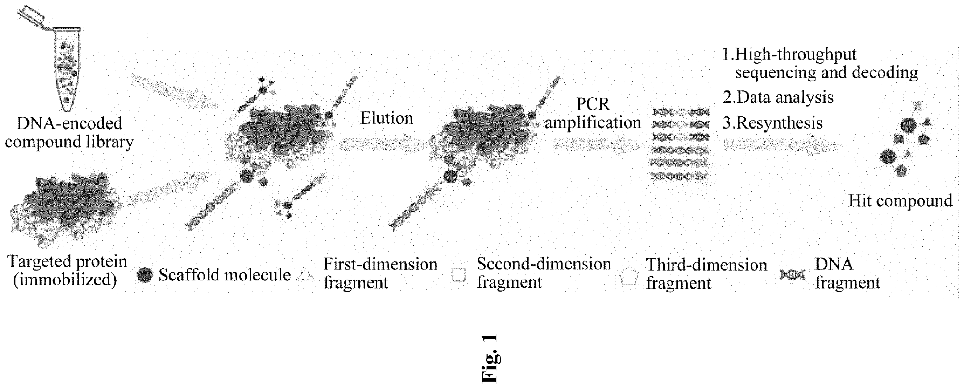

Traditionally, the drug screening in a DNA-encoded compound library is performed through incubation of the library with the target, separation of the compound bound to the target from other compounds by elution, dissociation of the compound bound to the target under protein denaturing conditions, QPCR amplification, DNA sequencing, and data analysis to obtain the chemical structure of the compound bound to the target protein (As shown in Fig. 1). However, the DNA-encoded compound screening is an affinity screening, which relies on the interaction force between the drug molecule and the target. By using this screening method, it is easy to find molecules with affinity to the target, but has some application limitations, mainly in the elution process required in the screening process. Molecules with low affinity (micromolar-millimolar) are easily separated from the target during the elution process, so that they cannot be enriched in signal and are thus neglected. This limits the screening effects of DNA-encoded compound libraries, especially for small molecule fragments during hybrid screening.

To solve the above technical problems, the present disclosure provides a DNA-encoded compound, a compound library and a screening method. The DNA-encoded compound library is incubated with the target, and the binding between the compound and the target is enhance by covalent cross-linking. Compared with the conventional affinity screening of the DNA-encoded compound library, the screening method provided herein can improves the discrimination of compounds, especially low-affinity compounds, on the screening signal.

### SUMMARY

In a first aspect, this application provides a DNA-encoded compound of formula (I): 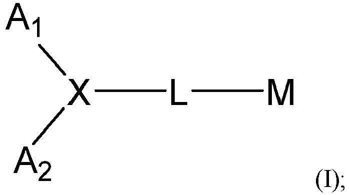 wherein X is an atomic or molecular scaffold;
A₁ is a first moiety comprising a first linker and a first oligonucleotide;
A₂ is a second moiety comprising a second linker and a second oligonucleotide;
L is a linker moiety comprising at least one group operable for covalent cross-linking;
M is a functional moiety comprising at least one structural unit.

In some embodiments, X is a carbon atom, a nitrogen atom, a cyclic scaffold or a non-cyclic scaffold.

In some embodiments, the DNA-encoded compound is represented by formula (II): 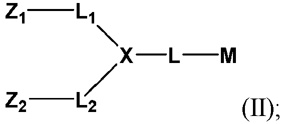
wherein Z₁ is the first oligonucleotide with its 3' terminus attached to L₁, and Z₂ is the second oligonucleotide with its 5' terminus attached to L₂; or Z₁ is the first oligonucleotide with its 5' terminus attached to L₁, and Z₂ is the second oligonucleotide with its 3' terminus attached to L₂;
L₁ is the first linker comprising a first functional group capable of forming a covalent bond with the 3' terminus or 5' terminus of Z₁; and
L₂ is the second linker comprising a second functional group capable of forming a covalent bond with the 5' terminus or 3' terminus of Z₂.

In some embodiments, Z₁ and Z₂ are at least partially complementary to each other to form a double-stranded structure; Z₁ and Z₂ each independently have a length of at least 10 bases and a complementary region of Z₁ and Z₂ has a length of at least 10 bases.

In some embodiments, Z₁ and Z₂ each independently has a polymerase chain reaction (PCR) primer sequence.

In some embodiments, L₁ and L₂ are independently an alkylidene chain or poly (ethylene glycol) chain containing two functional groups; and the two groups are each independently selected from the group consisting of a phosphate group, an amino group, a hydroxyl group, and a carboxyl group.

In some embodiments, L₁ and L₂ are independently 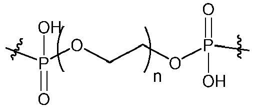 or 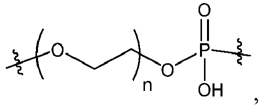 wherein n is an integer selected from 1 to 10, preferably, 3.

In some embodiments, the at least one group contained in L is a photosensitive group, an electrosensitive group, or other groups capable of forming covalent cross-linking with a protein.

In some embodiments, the at least one group contained in L is selected from the group consisting of an acridinyl group, an aryl azido group, a diphenyl ketone group, a sulfonyl fluoride group, an α,β-unsaturated acid group, an α,β-unsaturated ketone group, an α,β-unsaturated ester group, an α,β-unsaturated sulfonyl group, an α-acyl halide group, an epoxy group, an aldehyde group, a cyano group, and a boronic acid group.

In some embodiments, L has a structure of:

-S₁-S₂-S₃-;

wherein S₁ and S₃ are independently a cyclic or non-cyclic linker formed by carbon atoms, heteroatoms or a combination thereof and carrying at least one functional group, wherein the at least one functional group is each independently selected from the group consisting of a phosphate group, an amino group, a hydroxyl group, a carboxyl group, an aldehyde group, an azido group, an alkynyl group, and a halogen; and
S₂ is a linker containing the at least one group operable for covalent cross-linking.

In some embodiments, S₁ is connected to X, and S₃ is connected to M.

In some embodiments, S₃ is connected to X, and S₁ is connected to M.

In some embodiments, S₁ and S₃ are independently selected from the group consisting of 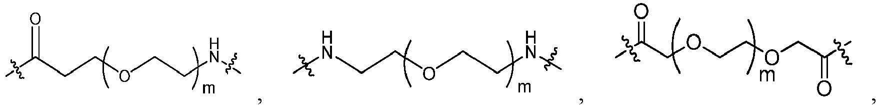 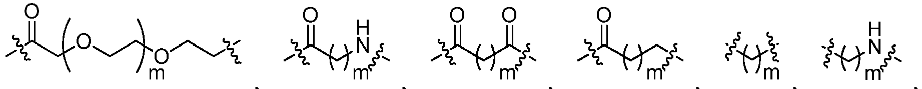 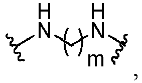 and a combination thereof, wherein m is a integer selected from 1 to 20. When the S₁ or S₃ is absent, it indicates that the two moieties connected thereto are directly linked through a covalent bond. Preferably, m is 1, 2, 3, 4, 5, 6, 7, 8, 9, or 10.

In some embodiments, the at least one group operable for covalent cross-linking is linked to S₁ and S₃, and is selected from the group consisting of: 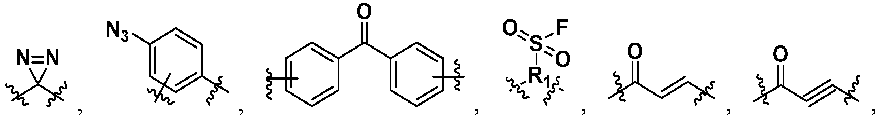 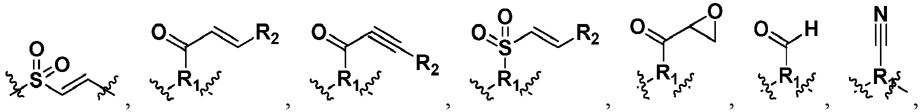 and 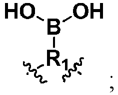 wherein R₁ is a carbon or nitrogen atom; and R₂ is hydrogen, an alkyl with or without a heteroatom, or an aryl with or without a heteroatom.

In some embodiment, S₂ is 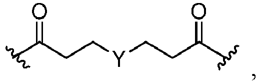 wherein Y is 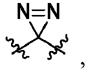 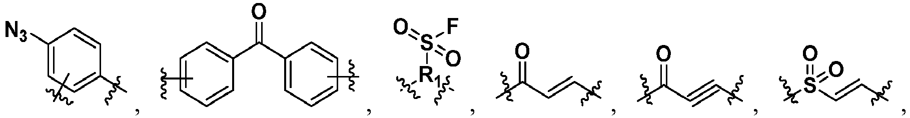 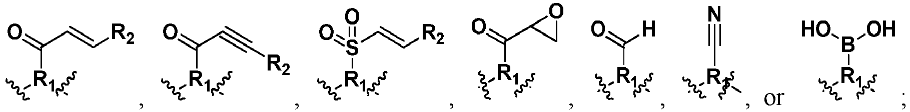 R₁ is a carbon atom or a nitrogen atom; and R₂ is hydrogen, an alkyl with or without a heteroatom, or an aryl with or without a heteroatom.

In some embodiments, S₂ is

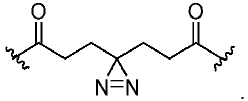

In some embodiments, the at least one group operable for covalent cross-linking is no more than 30 atoms away from R, preferably, no more than 15 atoms.

In some embodiments, the at least one group operable for covalent cross-linking is more than 4 atoms away from R, preferably, more than 5 atoms.

In a second aspect, this application provides a starting fragment compound for synthesizing a DNA-encoded compound library, wherein the starting fragment compound is represented by formula (III):

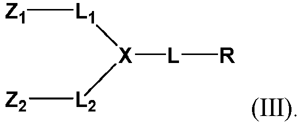

The starting fragment compound has a structure of: 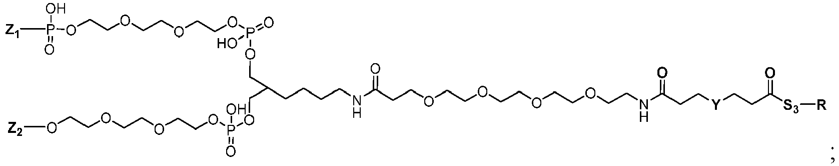 wherein Z₁ is a first oligonucleotide attached at its 3' terminus, and Z₂ is a second oligonucleotide attached at its 5' terminus; Y is 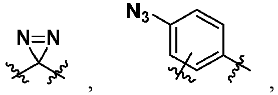 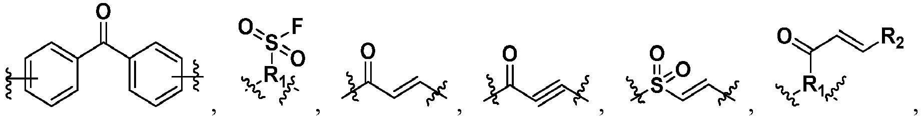 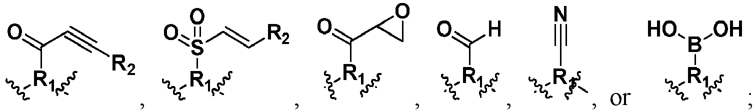 R₁ is a carbon atom or a nitrogen atom; R₂ is hydrogen, an alkyl with or without a heteroatom, or an aryl with or without a heteroatom; S₃ is selected from the group consisting of 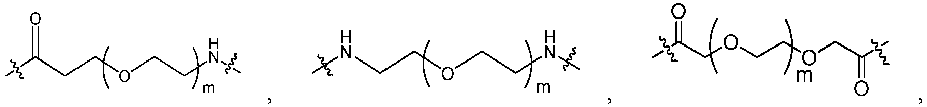 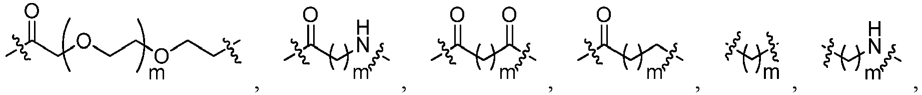 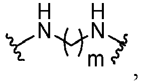 and a combination thereof, wherein m is a integer selected from 1 to 20. When S₃ is absent, it means that two parts connected thereto are directly linked through a covalent bond. Preferably, m is 1, 2, 3, 4, 5, 6, 7, 8, 9, or 10. R is a reactive group linking to a functional moiety, preferably, R is a phosphate group, an amino group, a hydroxyl group, a carboxyl group, or an aldehyde group.

In some embodiments, the starting fragment compound has a structure of: 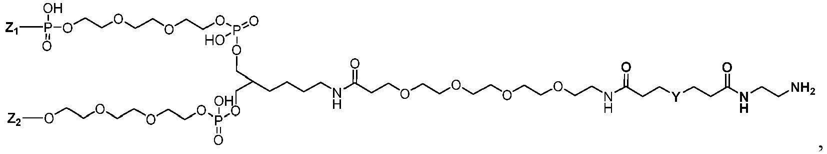 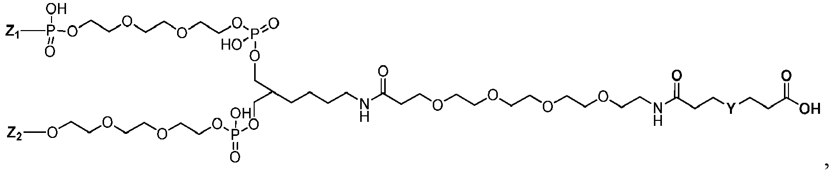 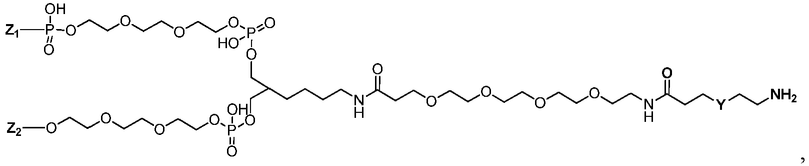 or 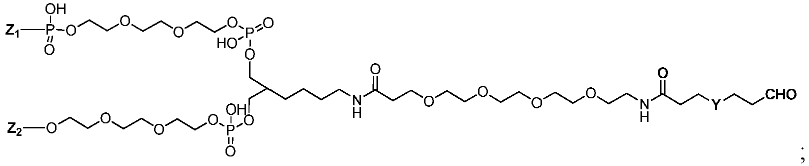 wherein Z₁ is a first oligonucleotide attached at its 3' terminus, and Z₂ is a second oligonucleotide attached at its 5' terminus; Y is 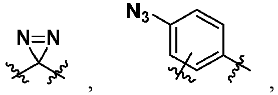 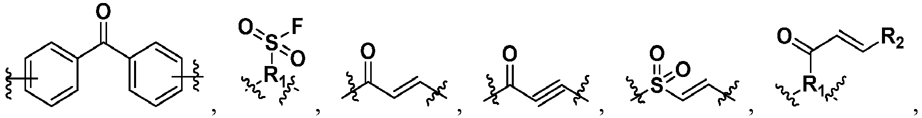 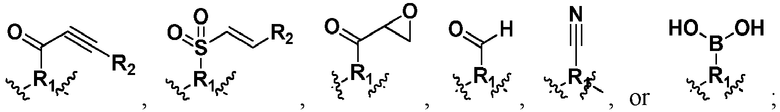 and R₁ is a carbon atom or a nitrogen atom; R₂ is hydrogen, an alkyl with or without a heteroatom, or an aryl with or without a heteroatom.

In a third aspect, this application provides a screening method for the aforementioned DNA-encoded compound library, comprising:
(S1) incubating the DNA-encoded compound library with a protein target, followed by covalent cross-linking to obtain a covalently cross-linked protein-DNA-encoded compound complex;
(S2) separating and recovering the covalently cross-linked protein-DNA-encoded compound complex from members in the DNA-encoded compound library that do not cross-link with the protein target; and
(S3) subjecting the covalently cross-linked protein-DNA-encoded compound complex to polymerase chain reaction (PCR) amplification and sequencing to read DNA sequence information and acquire compound structure information.

In some embodiments, in step (S 1), the covalent cross-linking is performed by irradiation, heating, electricity, or direct incubation.

In some embodiments, in step (S2), the separating is performed through steps of:
perform protein immobilization; and
eluting the members in the DNA-encoded compound library that do not cross-link with the protein target with an eluent.

In some embodiments, the protein immobilization is performed by using magnetic beads.

The screening method by using the DNA-encoded compound library provided herein can enhance the binding of compounds to targets by covalent cross-linking and thus improves the differentiation of screening signals for compounds, in particular low-affinity compounds and fragmented compounds.

The term "functional moiety" used herein refers to the small molecule part of the DNA-encoded compound library, which is used for screening with biological targets by means of combinatorial or non-combinatorial chemistry to build a diversity of molecular structures. The carbon atoms described herein can be freely chosen according to the understanding of those skilled in the art. For example, "CH₂" refers to divalent substitution, and "CH" refers to trivalent substitution.

The terms "molecular scaffold" and "scaffold structure" used herein can have different substitution sites according to the understanding of one of ordinary skill in the art. For example, "two substitution sites" refers to divalent substitution, and "three substitution sites" refers to trivalent substitution.

The term "oligonucleotides" used herein include, but are not limited to, DNA, RNA, PNA, and combinations thereof, of which the sequence information can be read by one of ordinary skill in the art by means of ordinary technical knowledge and customary means in the art.

The DNA-encoded compound libraries provided herein are suitable for the screening of a wide range of biological targets. One of ordinary skill in the art may select the different types of DNA-encoded compound libraries of the present disclosure as needed.

In the DNA-encoded compounds/libraries provided herein, the group capable for covalent cross-linking is directly linked to the linker strand, greatly reducing the non-specific covalent binding of covalent cross-linking groups.

According to the content of the present disclosure, one of ordinary skill in the art may select functional groups in the DNA-encoded compound and in the starting fragment compound of the encoded compound library that are suitable for pairing for linkage in accordance with the ordinary technical knowledge and the customary means in the art.

Obviously, modifications, substitutions or alterations can be made without departing from the above-mentioned basic technical idea of the invention in accordance with the ordinary technical knowledge and the customary means in the art.

The present disclosure will be described in detail below through specific embodiments. However, it should be understood that the scope of the above-mentioned subject matter of the present invention is not limited to the following examples. Any technology implemented on the basis of the above-mentioned contents of the present disclosure shall fall within the scope of the present disclosure.

### BRIEF DESCRIPTION OF THE DRAWINGS

Fig. 1 schematically depicts a conventional DNA-encoded compound library screening process;
Fig. 2 schematically shows chemical structures of four DNA-encoded compounds obtained by Example 1;
Fig. 3 schematically show chemical structures of four compounds in Example 1, whose Kd values with a CAIX target increase in sequence, that is, affinities of the four compounds decrease in sequence; where R1 is a fluorescent group for Kd value determination;

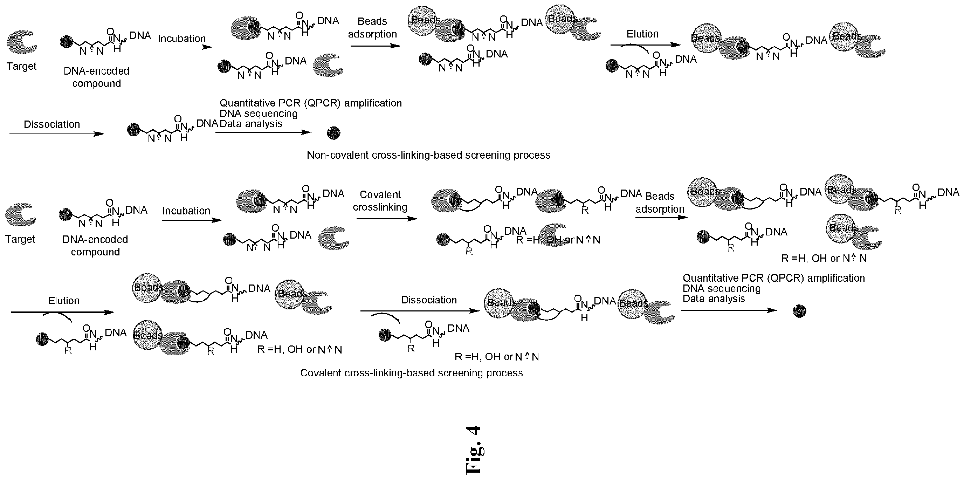

Fig. 4 schematically depicts a DNA-encoded compound screening process;
Fig. 5 graphically shows screening recovery results of a DNA-encoded compound in Example 1;
Fig. 6 graphically shows screening signal results of a DNA-encoded compound library in Example 2;
Fig. 7 schematically shows a structure of the DNA-encoded compound library constructed in Example 3; and
Figs. 8a-8d graphically show screening signal results of a DNA encoded compound library in Example 3.

### DETAILED DESCRIPTION OF EMBODIMENTS

The technical solutions of the present disclosure will be further described below with reference to the accompanying specific embodiments. Obviously, the described embodiments are only part of the embodiments of the present disclosure. The raw materials and equipment used herein are known products, and are purchased from commercially available products.

DNA-NH₂ or 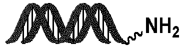 in the present disclosure is a DNA structure with a -NH₂ junction formed by single- or double-stranded DNA and a junction group, such as the DNA-NH₂ structure of "compound 1" described in PCT application WO2005058479, and the following DNA structures: 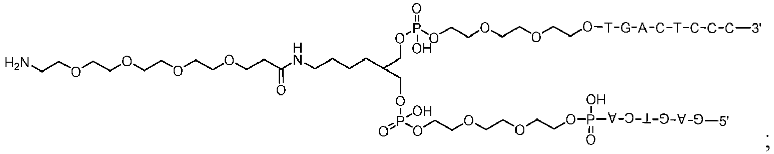 where A is adenine, T is thymine, C is cytosine, and G is guanine.

Other abbreviations used in this application are described as follows. Fmoc represents fluorenylmethoxycarbonyl. DMT-MM represents 2-chloro-4,6-dimethoxy-1,3,5-triazine. DIPEA represents N,N-diisopropylethylamine. DMA represents N,N-dimethylacetamide. HATU represents 2-(7-oxobenzotriazole)-N,N,N',N'-tetramethyluronium hexafluorophosphate.

### Embodiment 1 Synthesis and screening of a DNA-encoded compound

### Step (1) Synthesis of a DNA-encoded compound

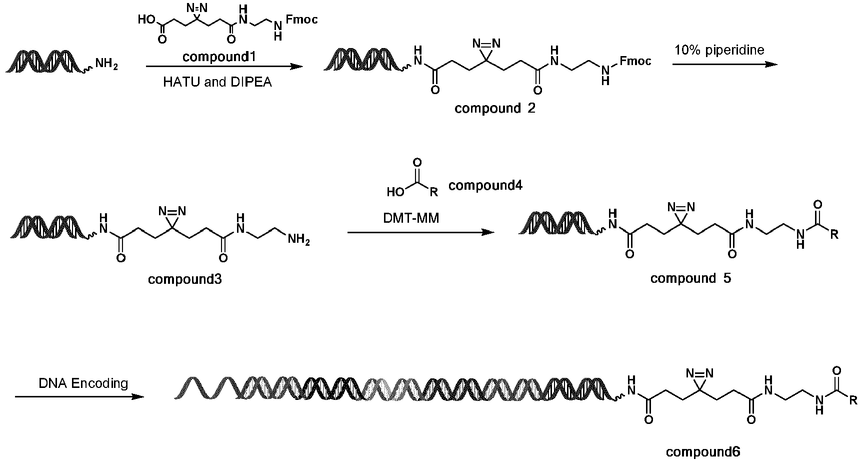

(1) DNA-NH₂ was dissolved in a borate buffer solution (250 mM, pH = 9.4) to form a first solution (1 mM). Compound 1 in a DMA solution (50 equiv., 200 mM), HATU in a DMA solution(50 equiv., 400 mM) and DIPEA in a DMA solution (100 equiv., 400 mM) were separately pre-chilled in a -20°C refrigerator for 5 minutes and mixed to form a mixture solution, and then the mixture solution was stored in a 4°C refrigerator for 5 min and added to the first solution to form a first reaction mixture, followed by shaking, mixing and standing for reaction at room temperature for 12 h.

After the reaction was completed, the first reaction mixture was subjected to ethanol precipitation. Specifically, 5M NaCl solution was added to the first reaction mixture, then anhydrous ethanol was added, where the 5M NaCl solution was 10% of a total volume of the first reaction mixture, and the anhydrous ethanol was 3 times the total volume of the first reaction mixture. After uniform mixing by shaking, the first reaction mixture was placed on dry ice and frozen for 2 h, followed by centrifugation at 12,000 rpm for 0.5 h. After that, the supernatant was poured off and the remaining precipitate was dissolved in deionized water to obtain the crude product of compound 2, which was directly used in the subsequent reaction without purification.

(2) The crude product of compound 2 was dissolved in pure water to form a second solution (1 mM). Piperidine was added into the second solution to form a second reaction mixture followed by vortex oscillation and standing for reaction at room temperature for 1-3 h, where the piperidine was 10% of the total volume of the second solution.

After the reaction was completed, the second reaction mixture was subjected to ethanol precipitation. Specifically, 5M NaCl solution was added to the second reaction mixture, then anhydrous ethanol was added, where the 5M NaCl solution was 10% of a total volume of the second reaction mixture, and the anhydrous ethanol was 3 times the total volume of the second reaction mixture. After uniform mixing by shaking, the second reaction mixture was placed on dry ice and frozen for 2 h, followed by centrifugation at 12,000 rpm for 0.5 h. After that, the supernatant was poured off and the remaining precipitate was dissolved in deionized water to obtain the crude product of compound 3, which was directly used in the subsequent reaction without purification.

(3) The crude product of compound 3 was dissolved in a borate buffer solution (250 mM, pH = 9.4) to form a third solution (1 mM), and then sequentially added with a carboxylic acid compound 4 in a DMA solution (100 equiv., 100 mM) and a DMT-MM in deionized water (100 equiv., 100 mM) to form a third reaction mixture. The third reaction mixture was uniformed mixed by vortex oscillation and then standing for reaction at room temperature for 12-16 h.

After the reaction was completed, the third reaction mixture was subjected to ethanol precipitation. Specifically, 5M NaCl solution was added to the third reaction mixture, then anhydrous ethanol was added, where the 5M NaCl solution was 10% of a total volume of the third reaction mixture, and the anhydrous ethanol was 3 times the total volume of the third reaction mixture. After uniform mixing by shaking, the third reaction mixture was placed on dry ice and frozen for 2 h, followed by centrifugation at 12,000 rpm for 0.5 h. After that, the supernatant was poured off and the remaining precipitate was dissolved in deionized water to obtain the crude product of compound 5, which was purified by preparative chromatography to obtain a purified compound 5.

(4) 100 nmol of the purified compound 5 was dissolved in pure water to obtain a fourth solution (1 mM, 100 µL). Primer 1 in water (166.6 nmol, 1.67 equiv., 2 mM, 83.3 µL), 10 of ligation buffer (66.6 µL), T4 DNA ligase (9.6 µL, 13.97 µg/µL) and deionized water (407 µL) were added into the fourth solution to obtain a fourth reaction mixture followed by vortex oscillation and standing for reaction at 20°C for 16 h.

After the reaction was completed, the fourth reaction mixture was subjected to ethanol precipitation. Specifically, 5M NaCl solution was added to the fourth reaction mixture, then anhydrous ethanol was added, where the 5M NaCl solution was 10% of a total volume of the fourth reaction mixture, and the anhydrous ethanol was 3 times the total volume of the fourth reaction mixture. After uniform mixing by shaking, the fourth reaction mixture was placed on dry ice and frozen for 2 h, followed by centrifugation at 12,000 rpm for 0.5 h. After that, the supernatant was poured off and the remaining precipitate was dissolved in deionized water to obtain the crude product.

The crude product was dissolved in pure water to form a fifth solution (1 mM, 100 µL). The fifth solution was added with ligated DNA fragment 1-DNA fragment 2-DNA fragment 3-primer 2-library ID in water (100 nmol, 1 equiv., 2 mM, 50 µL), 10 of ligation buffer (80 µL), T4 DNA ligase (4.31 µL, 13.97 µg/µL) and deionized water (165 µL) to form a fifth reaction mixture followed by vortex oscillation and standing for reaction at 20°C for 16 h.

After the reaction was completed, the fifth reaction mixture was subjected to ethanol precipitation. Specifically, 5M NaCl solution was added to the fifth reaction mixture, then anhydrous ethanol was added, where the 5M NaCl solution was 10% of a total volume of the fifth reaction mixture, and the anhydrous ethanol was 3 times the total volume of the fifth reaction mixture. After uniform mixing by shaking, the fifth reaction mixture was placed on dry ice and frozen for 2 h, followed by centrifugation at 12,000 rpm for 0.5 h. After that, the supernatant was poured off and the remaining precipitate was dissolved in deionized water to obtain the compound 6.

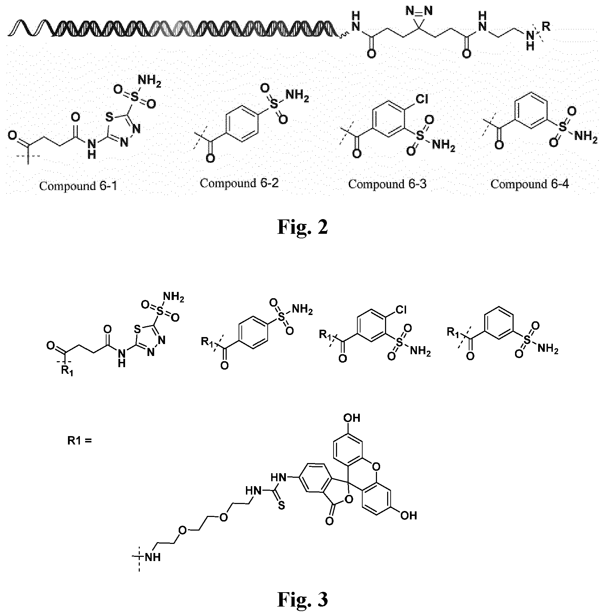

Four compounds 6 (i.e., compound 6-1, compound 6-2, compound 6-3 and compound 6-4) were synthesized according to the above synthesis method, and their specific structures were shown in Fig. 2.

### Step (2) Screening of a DNA-encoded compound

A DNA-encoded compound (0.2 nM) and 100 pmol of a target protein were added into a screening buffer (the screening buffer consisted of 12.5 mM Tris, 150 mM NaCl, 0.3 mg/mL ssDNA, and 0.05% Tween20, and had a pH of 7.5) having a total volume of 100 µL in a 1.5 mL centrifuge tube. In the meanwhile, a parallel blank control group (no target protein was added) was set up, and each of the target protein group and the blank control group was made for 2 groups. The centrifuge tubes containing reaction mixture were place on a rotary mixer for incubation at 20 rpm and 25°C for 60 min. Two groups of incubated samples (one for the target protein group and the other for the blank control group) were placed on ice and exposed to 365 nm UV light for the photocrosslinking reaction for 10 min, while the other two groups of samples (one for the target protein group and the other for the blank control group) were placed on ice for 10 min without exposure to the 365 nm UV light.

Then 25 µL of Ni-charged magnetic beads, which were equilibrated with 250 µL of a screening buffer three times, were put into the UV-lighted or non-lighted samples. Then the centrifuge tubes were placed on the rotary mixer for incubation at 20 rpm and 25°C for 30 min. By the affinity of the target protein tag with the specific magnetic beads, the target protein and the target protein-bound DNA-encoded complex were separated from the solution with a magnetic holder, and the supernatant was collected.

The separated magnetic beads were re-suspended with 500 µL of the screening buffer and washed on the rotary mixer at 20 rpm and 25°C for 1min to wash off the non-specifically bound DNA-encoded compound, and the magnetic holder was used to separate the magnetic beads from the supernatant. These processes were repeated 5 times.

The washed magnetic beads were re-suspended with 100 µL of an elution buffer (the elution buffer consisted of 12.5 mM Tris and 150 mM NaCl and had a pH of 7.5), and eluted in a metal bath at 95°C for 10 min. The magnetic beads were separated from the eluted DNA-encoded compound with the magnetic stand. Then, the samples on the magnetic beads were the DNA-encoded compound samples obtained by photocrosslinking, and the elution supernatant was the DNA-encoded compound sample obtained by affinity interaction.

The elution supernatant and magnetic beads were each diluted 20-fold with a dilution buffer (the dilution buffer consisted of 10 mM Tris and 0.05% Tween20, and had a pH of 8.0), and the DNA-encoded compound was also diluted 100-fold. A sample, a primer, deionized water and a qPCR mix (ABI, A25778) were configured into a 20 µL reaction solution for qPCR testing. The qPCR tests were performed under the following conditions: the pre-denaturation was performed at 95°C for 10 min; 35 cycles were performed (each cycle included denaturation at 95°C for 10 s, annealing at 55°C for 10 s and extension at 72°C for 10 s); and the signal collection was set at the extension step. After the qPCR tests were completed, the molecular copy number of samples was calculated according to Avogadro's constant and the molecular copy number differences between the samples were analyzed.

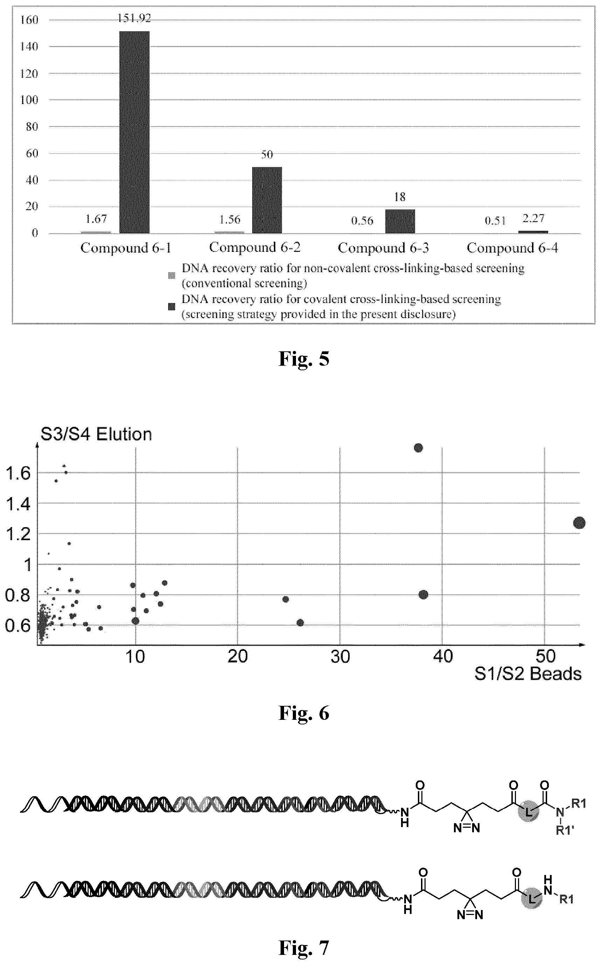

The screening results were shown in Fig. 5. The test results showed that under the screening conditions (non-light conditions) for the conventional DNA-encoded compound library, the DNA recovery ratio was 0.51-1.67 and there was no significant differentiation in the screening signal. By using the DNA-encoded compound library and screening method of the present disclosure, the DNA recovery ratio was 2.27-151.92, the differentiation in the screening signal was significantly increased, and the recovery ratio was in a positive correlation to the compound activity (as shown in Fig. 3).

### Embodiment 2 Synthesis of a DNA-encoded compound and screening for a phosphatase target

By using the starting DNA material (compound 3) prepared in Example 1, a DNA-encoded compound library containing 962 compounds was constructed according to the method described in the international patent application WO2005058479, represented by:

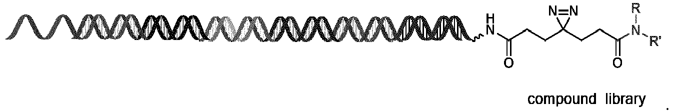

The screening was performed on the DNA-encoded compound library through the following steps.
(1) The DNA-encoded compound library (5.65 nM) and 250 pmol of a target protein were added into a screening buffer (the screening buffer consisted of 50 mM HEPES, 150 mM NaCl, 0.01% Tween-20, 0.3 mg/mL ssDNA, 10 mM imidazole, and had a pH of 7.4) having a total volume of 100 µL in a 1.5 mL centrifuge tube. In the meanwhile, a parallel blank control group (no target protein was added) was set up, and each of the target protein group and the blank control group was made for 2 groups. The centrifuge tubes containing reaction mixture were placed on a rotary mixer for incubation at 20 rpm and 25°C for 60 min. Two groups of incubated samples (one for the target protein group and the other one for the blank control group) were placed on ice and exposed to 365 nm UV light for the photocrosslinking reaction for 10 min, while the other two groups of samples (one for the target protein group and the other one for the blank control group) were placed on ice for 10 min without exposure to the 365 nm UV light.
(2) Then 20 µL of Ni-charged magnetic beads, which were equilibrated with 200 µL of a screening buffer three times, were put into the UV-lighted or non-lighted samples. Then the centrifuge tubes were placed on the rotary mixer for incubation at 20 rpm and 25°C for 30 min. By the affinity of the target protein tag with the specific magnetic beads, the target protein and the target protein-bound DNA-encoded complex were separated from the solution with a magnetic holder, and the supernatant was collected.
(3) The separated magnetic beads were re-suspended with 500 µL of the screening buffer and washed on the rotary mixer at 20 rpm and 25°C for 1 min to wash off the non-specifically bound DNA-encoded compound, and the magnetic holder was used to separate the magnetic beads from the supernatant. These processes were repeated 5 times.
(4) The washed magnetic beads were re-suspended with 55 µL of an elution buffer (the elution buffer consisted of 50 mM HEPES, 300 mM NaCl and had a pH of 7.4), and eluted in a metal bath at 95°C for 10 min. The magnetic beads were separated from the eluted DNA-encoded compound with the magnetic stand. Then, the samples on the magnetic beads were the DNA-encoded compound samples obtained by photocrosslinking, and the elution supernatant was the DNA-encoded compound sample obtained by affinity interaction.
(5) The elution supernatant and magnetic beads were each diluted 20-fold with a dilution buffer (the dilution buffer consisted of 10 mM Tris and 0.05% Tween20, and had a pH of 8.0), and the DNA-encoded compound was also diluted 100-fold. A sample, a primer, deionized water and a qPCR mix (ABI, A25778) were configured into a 20 µL reaction solution for qPCR testing. The qPCR tests were performed under the following conditions: the pre-denaturation was performed at 95°C for 10 min; 35 cycles were performed (each cycle included denaturation at 95°C for 10 s, annealing at 55°C for 10 s and extension at 72°C for 10 s); and the signal collection was set at the extension step. After the qPCR tests were completed, the molecular copy number of samples was calculated according to Avogadro's constant.

The screening results were shown in Fig. 6, where the S3/S4 Elution on the vertical coordinate represented the screening signal result of the conventional DNA-encoded compound library, and the vertical coordinate value represented the DNA recovery ratio, which showed that the DNA recovery ratios for all compounds were 0-2, with no significant signal differentiation. The S 1/S3 Beads on the horizontal coordinate showed the screening results of the DNA-encoded compound library provided in this application, and the vertical coordinate value represented the DNA recovery ratios, which indicated that the DNA recovery ratios of the compounds were 0-60, with significant signal differentiation.

### Embodiment 3 Synthesis of a DNA-encoded compound and screening for a kinase target

A DNA-encoded compound library containing 7417 compounds as shown in Fig. 7 was constructed with reference to the method for constructing a DNA-encoded compound library described in Example 1 of the present disclosure and the international patent application WO2005058479, where L was a linker chain different in lengths.

The kinase target PAK4 was screened using the DNA-encoded compound library provided in this embodiment with reference to the screening method described in Example 2 of this application. The screening groupings were shown in Table 1, where "+" indicated that the condition was present, and "-" indicated that the condition was absent.

**Table 1 Screening groupings**

| | PAK4 protein | UV Irradiation | Competitive molecule | Control protein |
|---|---|---|---|---|
| Target-free screening control group | - | + | - | - |
| PAK4-irradiation group | + | + | - | - |
| PAK4 irradiation-free group | + | - | - | - |
| PAK4 irradiation group in the presence of a competitive molecule-added | + | + | + | - |
| Irradiation group in the presence of another target | - | + | - | + |

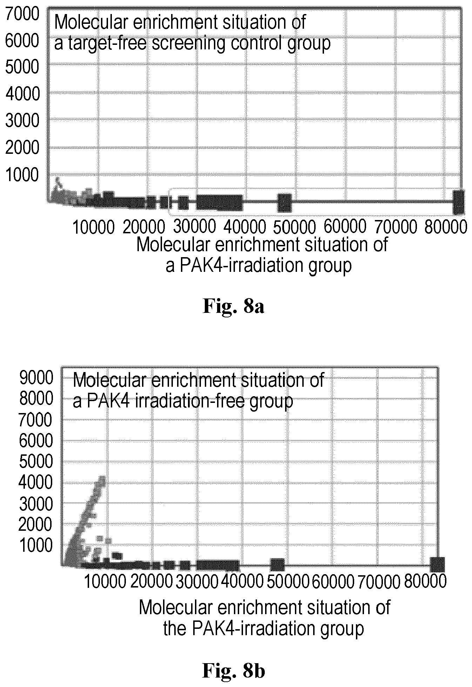

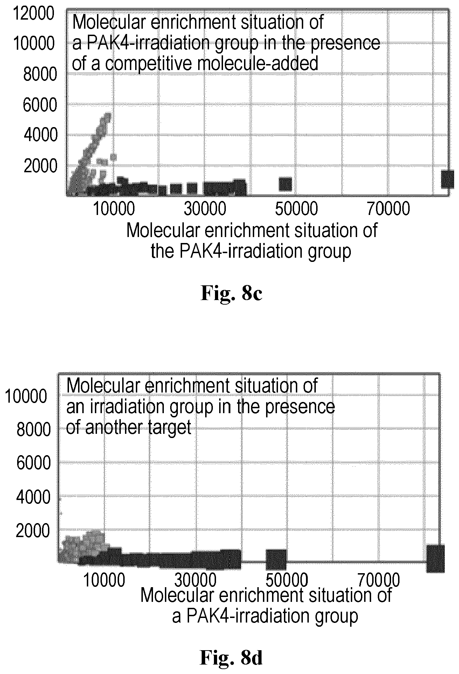

The results of the screening signals were shown in Figs. 8a-8d, where the horizontal and vertical coordinates showed the enrichment of the screening signals (molecular enrichment) for the different screening groups, and the values indicated the degree of enrichment. Figs. 8a-8d showed that the compound library and the screening method of the present disclosure reached a good signal enrichment. In addition, Fig. 8a showed that the molecules were not enriched on materials other than proteins (e.g., magnetic beads). Fig. 8b showed that the enrichment of the molecules under light covalent cross-linking was much higher than the enrichment of the molecules under non-light conditions. Fig. 8c showed that the signal intensity of the molecules was reduced by competitive molecules, indicating that they acted on the correct target pocket. Fig. 8d showed that the molecules did not suffer from severe non-specific binding.

The experimental results show that by using the DNA encoded compounds/library and screening method of the present disclosure, the binding between the compound and the target can be enhanced, the discrimination of compounds, particularly low affinity compounds, on the screening signal can be improved, producing positive results.

In summary, the present disclosure provides a method for the synthesis of DNA-encoded compounds and compound libraries, and a screening method thereof. In the present disclosure, the DNA-encoded compound library is incubated with a target site followed by covalent cross-linking to enhance the binding of the compound to the target site. Compared with the conventional affinity screening of DNA-encoded compound libraries, it can improve the discrimination of compounds, particularly low affinity compounds, on the screening signal.

## Claims

1. A DNA-encoded compound of formula (I): 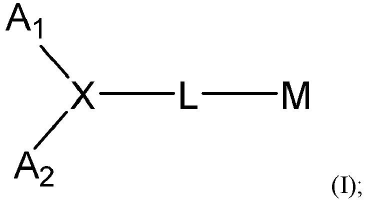
**characterized in that** X is an atomic or molecular scaffold;
A₁ is a first moiety comprising a first linker and a first oligonucleotide;
A₂ is a second moiety comprising a second linker and a second oligonucleotide;
L is a linker moiety comprising at least one group operable for covalent cross-linking;
M is a functional moiety comprising at least one structural unit.

2. The DNA-encoded compound according to claim 1, **characterized in that** X is a carbon atom, a nitrogen atom, a cyclic scaffold or a non-cyclic scaffold.

3. The DNA-encoded compound according to claim 2, **characterized in that** the DNA-encoded compound is represented by formula (II): 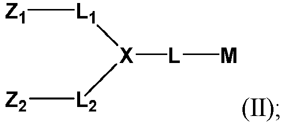
wherein Z₁ is the first oligonucleotide with its 3' terminus attached to L₁, and Z₂ is the second oligonucleotide with its 5' terminus attached to L₂; or Z₁ is the first oligonucleotide with its 5' terminus attached to L₁, and Z₂ is the second oligonucleotide with its 3' terminus attached to L₂;
L₁ is the first linker comprising a first functional group capable of forming a covalent bond with the 3' terminus or 5' terminus of Z₁; and
L₂ is the second linker comprising a second functional group capable of forming a covalent bond with the 5' terminus or 3' terminus of Z₂.

4. The DNA-encoded compound according to claim 3, **characterized in that** Z₁ and Z₂ are at least partially complementary to each other to form a double-stranded structure; Z₁ and Z₂ each independently have a length of at least 10 bases, and a complementary region of Z₁ and Z₂ has a length of at least 10 bases.

5. The DNA-encoded compound according to claim 4, **characterized in that** Z₁ and Z₂ each independently has a polymerase chain reaction (PCR) primer sequence.

6. The DNA-encoded compound according to claim 3, **characterized in that** L₁ and L₂ are independently an alkylene chain or poly(ethylene glycol) chain containing two functional groups, wherein the two functional groups are each independently selected from the group consisting of a phosphate group, an amino group, a hydroxyl group, and a carboxyl group.

7. The DNA-encoded compound according to claim 6, **characterized in that** L₁ and L₂ are independently 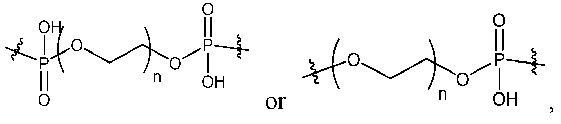 wherein n is an integer selected from 1 to 10.

8. The DNA-encoded compound according to claim 3, **characterized in that** the at least one group contained in L is a photosensitive group, an electrosensitive group, or other groups capable of forming covalent cross-linking with a protein.

9. The DNA-encoded compound according to claim 8, **characterized in that** the at least one group contained in L is selected from the group consisting of an acridinyl group, an aryl azido group, a diphenyl ketone group, a sulfonyl fluoride group, an α,β-unsaturated acid group, an α,β-unsaturated ketone group, an α,β-unsaturated ester group, an α,β-unsaturated sulfonyl group, an α-acyl halide group, an epoxy group, an aldehyde group, a cyano group, and a boronic acid group.

10. The DNA-encoded compound according to claim 3, **characterized in that** L has a structure of:
-S₁-S₂-S₃-;
wherein S₁ and S₃ are independently a cyclic or non-cyclic linker formed by carbon atoms, heteroatoms or a combination thereof and carrying at least one functional group, wherein the at least one functional group is each independently selected from the group consisting of a phosphate group, an amino group, a hydroxyl group, a carboxyl group, an aldehyde group, an azido group, an alkynyl group, and a halogen; and
S₂ is a linker containing the at least one group operable for covalent cross-linking.

11. The DNA-encoded compound according to claim 10, **characterized in that** S₁ and S₃ are independently selected from the group consisting of 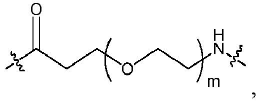 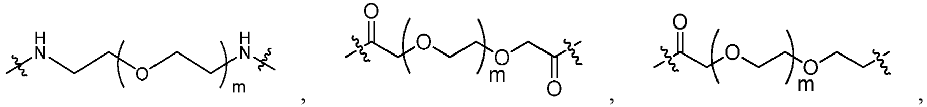 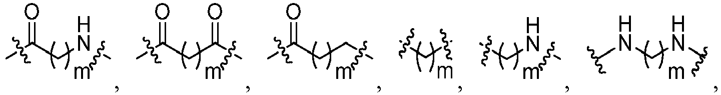 and a combination thereof, wherein m is a integer selected from 1 to 20.

12. The DNA-encoded compound according to claim 10, **characterized in that** the at least one group operable for covalent cross-linking is linked to S₁ and S₃, and is selected from the group consisting of: 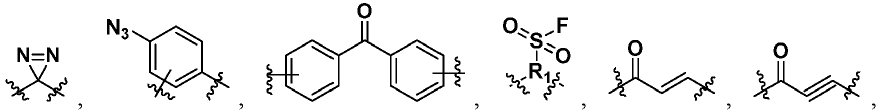 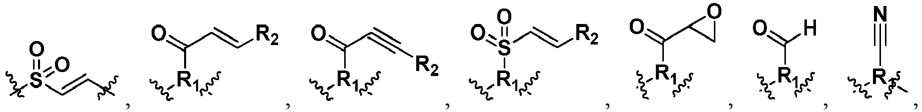 and 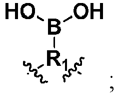 wherein R₁ is a carbon or nitrogen atom; and R₂ is hydrogen, an alkyl with or without a heteroatom, or an aryl with or without a heteroatom.

13. The DNA-encoded compound according to claim 12, **characterized in that** the at least one group operable for covalent cross-linking is no more than 15 atoms away from the functional moiety M.

14. The DNA-encoded compound according to claim 3, **characterized in that** X is 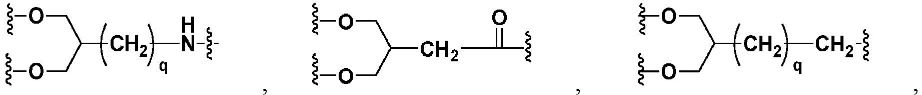 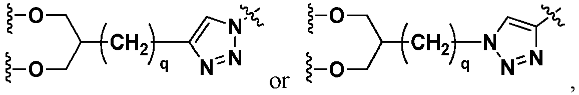 wherein q is an integer selected from 1 to 10.

15. A DNA-encoded compound library, **characterized in that** the DNA-encoded compound library consists of the DNA-encoded compound according to any one of claims 1-14.

16. The DNA-encoded compound library according to claim 15, **characterized in that** a total of at least 10² DNA-encoded compounds are contained in the DNA-encoded compound library.

17. A starting fragment compound for synthesizing a DNA-encoded compound library, **characterized in that** the starting fragment compound is represented by formula (III): 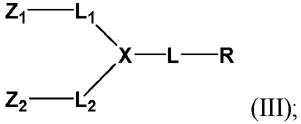
wherein X is an atomic or molecular scaffold;
Z₁ is a first oligonucleotide with its 3' terminus attached to L₁, and Z₂ is a second oligonucleotide with its 5' terminus attached to L₂; or Z₁ is a first oligonucleotide with its 5' terminus attached to L₁, and Z₂ is a second oligonucleotide with its 3' terminus attached to L₂;
L₁ is a first linker comprising a first functional group capable of forming a covalent bond with the 3' terminus or 5' terminus of Z₁;
L₂ is a second linker comprising a first functional group capable of forming a covalent bond with the 5' terminus or 3' terminus of Z₂;
L is a linker moiety comprising at least one group operable for covalent cross-linking; and
R is a reactive group linked to a functional moiety.

18. The starting fragment compound according to claim 17, **characterized in that** X is a carbon atom, a nitrogen atom, a cyclic scaffold or a non-cyclic scaffold;
Z₁ and Z₂ are at least partially complementary to each other to form a double-stranded structure; and Z₁ and Z₂ each independently has a length of 5-15 bases;
L₁ and L₂ are independently an alkylene chain or poly (ethylene glycol) chain containing two functional groups, wherein the two functional groups are each independently selected from the group consisting of a phosphate group, an amino group, a hydroxyl group, and a carboxyl group;
the at least one group contained in L is a photosensitive group, an electrosensitive group, or other groups capable of forming covalent cross-linking with a protein; and
R is a phosphate group, an amino group, a hydroxyl group, a carboxyl group, or an aldehyde group.

19. The starting fragment compound according to claim 18, **characterized in that** X is 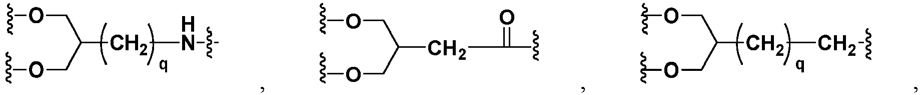 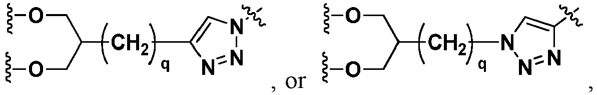 wherein q is an integer selected from 1 to 10;
L₁ and L₂ are independently 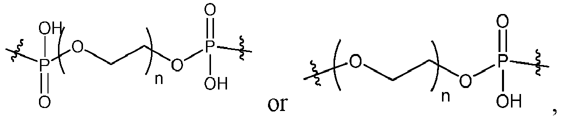 wherein n is an integer selected from 1 to 10; and
L has a structure of -S₁-S₂-S₃-, wherein S₁ and S₃ are independently 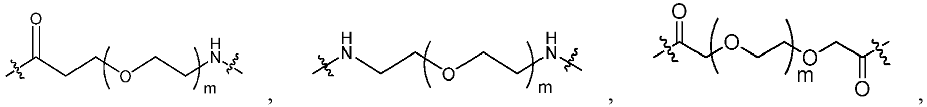 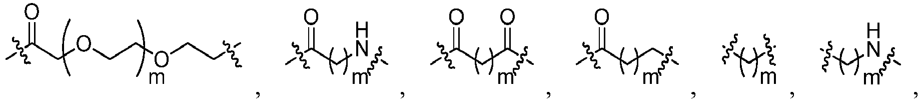 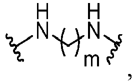 or a combination thereof, or absent; wherein m is an integer selected from 1 to 10; and S₂ is a linker containing the at least one group operable for covalent cross-linking.

20. The starting fragment compound according to claim 19, **characterized in that** the starting fragment compound is selected from the group consisting of: 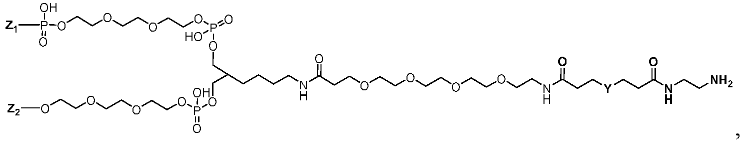 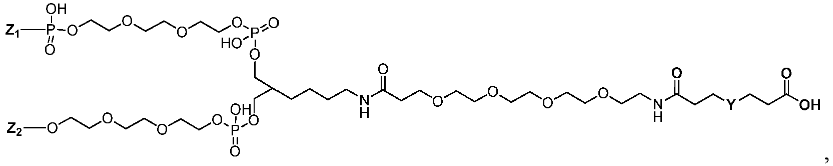 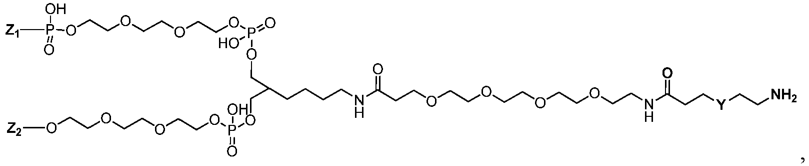 and 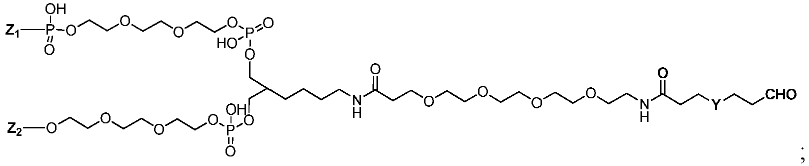 wherein Y is 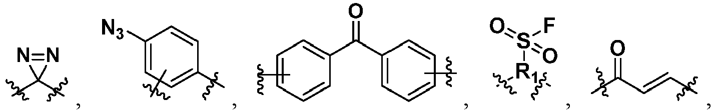 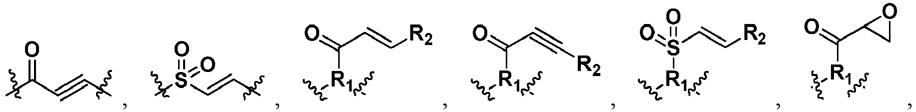 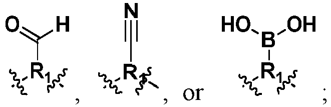 R₁ is a carbon atom or a nitrogen atom; and R₂ is hydrogen, an alkyl with or without a heteroatom, or an aryl with or without a heteroatom.

21. A screening method for the DNA-encoded compound library according to any one of claims 15-16, comprising:
(S1) incubating the DNA-encoded compound library with a protein target, followed by covalent cross-linking to obtain a covalently cross-linked protein-DNA-encoded compound complex;
(S2) separating the covalently cross-linked protein-DNA-encoded compound complex from members in the DNA-encoded compound library that do not cross-link with the protein target; and
(S3) subjecting the covalently cross-linked protein-DNA-encoded compound complex to polymerase chain reaction (PCR) amplification and sequencing to read DNA sequence information and acquire compound structure information.

22. The screening method according to claim 21, **characterized in that** in step (S1), the covalent cross-linking is performed by irradiation, heating, electricity, or direct incubation.

23. The screening method according to claim 21, **characterized in that** in step (S2), the separating is performed through steps of:
perform protein immobilization; and
eluting the members in the DNA-encoded compound library that do not cross-link with the protein target with an eluent.

24. The screening method according to claim 23, **characterized in that** the protein immobilization is performed by using magnetic beads.
